# EUROPEAN PATENT SPECIFICATION

(11) **EP 1 737 436 B1**
(45) Date of publication and mention of the grant of the patent: **07.07.2010**
(21) Application number: 05754644.2
(22) Date of filing: 27.05.2005
(51) Int. Cl.: A61K 31/16, A61K 31/655, A61P 1/04

(54) **PREVENTION, TREATMENT, AND AMELIORATION OF RADIATION INDUCED ENTERITIS**
PRÄVENTION, BEHANDLUNG UND LINDERUNG STRAHLUNGSINDUZIERTER ENTERITIS
PREVENTION, TRAITEMENT ET AMELIORATION DE L'ENTERITE RADIO-INDUITE

(30) Priority: 28.05.2004 US 608591 P
(43) Date of publication of application: 03.01.2007
(73) Proprietor: Salix Pharmaceuticals, Inc., Morrisville, NC 27560 (US)
(72) Inventor: BETTENHAUSEN, Doug, Raleigh, NC 27615-1848 (US); JAHRAUS, Christopher, Lexington-Fayette, KY 40509-2929 (US)
(74) Representative: Adam, Holger
(86) International application number: PCT/US2005/018757
(87) International publication number: WO 2005/117851

(56) References cited:
- US-A- 5 498 608
- US-A- 5 905 073
- US-A1- 2002 049 186
- US-A1- 2003 078 205
- US-B1- 6 197 341
- JAHRAUS C D ET AL: "Randomized double-blind placebo-controlled trial of balsalazide in the prevention of acute radiation enteritis as a consequence of pelvic radiotherapy" INTERNATIONAL JOURNAL OF RADIATION: ONCOLOGY BIOLOGY PHYSICS, PERGAMON PRESS, US, vol. 60, no. 1, September 2004 (2004-09), pages S253-S254, XP004557523 ISSN: 0360-3016
- JAHRAUS, C. ET AL.: "Prevention of acute radiation enteritis in patients receiving radiotherapy for prostate cancer: early results of a randomized double-blind placebo-controlled trial of balsalazide" AMERICAN JOURNAL OF GASTROENTEROLOGY, vol. 99, no. 10, October 2004 (2004-10), page S284-S285, XP009089780
- RAGUNATH, K . & WILLIAMS J.G.: "Review article: balsalazide therapy in ucerative colitis" ALIMENT PHARMACOL THER, vol. 15, 2001, pages 1549-1554, XP002451952

## Description

### RELATED APPLICATIONS

This application claims priority from U.S. Application No. 10/856,348, filed on May 28, 2004, which was converted into U.S. Provisional Application No. 60/608,951 on October 26, 2004.

### FIELD OF THE INVENTION

This invention relates to the use of balsalazide to treat, prevent, or ameliorate enteritis. More specifically, this invention relates to the use of balsalazide to treat radiation induced enteritis caused by radiation therapy, alone or in combination with other therapies.

### BACKGROUND OF THE INVENTION

Various pelvic therapies, including radiation therapy, chemotherapy, and surgical procedures, (sometimes referred to as "pelvic therapies"), which are used in a wide variety of clinical settings as either adjuvant or primary treatment for subjects with pelvic disorders, e.g., tumors, may cause bowel toxicity and other side effects such as acute radiation enteritis. For example, approximately 80% of patients undergoing pelvic radiotherapy experience acute radiation enteritis.

Side effects of these various pelvic therapies cause discomfort and may lead to a decrease in the therapeutic benefit of treatments because of the need for unscheduled breaks in therapy. Thus, it would be beneficial to have a treatment that prevents, ameliorates, or otherwise treats the side effects of pelvic therapies.

US 2003/0078205 discloses the use of an intestinal trefoil peptide in the treatment and prevention of a lesion of the distal bowel.

In an embodiment a second therapeutic agent may be added to the intestinal trefoil peptide. Said second therapeutic agent may be chosen among a great variety of compounds, and may be balsalazide.

US 2002/0049186 discloses the treatment of inflammatory conditions of the gastrointestinal tract with specific compounds which can be added a second therapeutic agent. Said second therapeutic agent may be chosen among a great variety of compounds, and may be balsalazide.

### SUMMARY OF THE INVENTION

Disclosed herein are uses for preventing, ameliorating and/or treating radiation induced enteritis. Also disclosed are uses for treating radiation induced enteritis caused by a combination of radiation therapy with chemotherapy and/or surgical procedures. In general, subjects who may benefit from treatment with balasalazide include those who are scheduled to begin or those who are in the process of undergoing radiation therapy, particularly in the pelvic region. Subjects who may particularly benefit from this treatment include those who are or may be susceptible to radiation induced enteritis. For example, the subjects may be about to undergo, may be undergoing, or may have undergone radiation therapy. The subjects may have also had a combination of pelvic therapies. Subjects may be suffering from, for example, gastrointestinal malignancies, including colorectal, appendiceal, anal, or small bowel cancers; urogenital malignancies, including prostate, bladder, testicular, or penile cancers; gynecologic malignancies, including cervical, endometrial, ovarian, vaginal, or vulvar cancers; or osteogenic and other sarcomatous malignancies in which pelvic structures are involved.

The present invention provides a new treatment for radiation induced enteritis.

According to one aspect of the present invention, the use for treating radiation induced enteritis comprises administering to a subject in need of such treatment a therapeutically effective amount of balsalazide.

In certain embodiments, the balsalazide is administered at least one day prior to the subject's first dose of radiotherapy. In a related embodiment, the balsalazide is administered at least five days prior to the subject's first dose of radiotherapy.

In certain other embodiments, the balsalazide is administered from at least one day prior to the first dose of radiotherapy until at least one day after the cessation of radiation therapy.

According to certain preferred embodiments, the balsalazide is administered twice daily to the subject.

In another embodiment, from between about 3,000 mg to about 5000 mg of balsalazide is administered daily to a subject.

In certain embodiments, the balsalazide is administered at least one day prior to a subject's first dose of radiotherapy, chemotherapy, or prior to undergoing a surgical procedure.

Certain other embodiment include the balsalazide being administered at least five days prior to a subject's first dose of radiotherapy, chemotherapy, and/or prior to undergoing a surgical procedure.

Other embodiments include balsalazide being administered during radiation therapy, chemotherapy, or the surgical procedure.

In certain preferred embodiments, the balsalazide is administered from at least one day prior to the administration of a pelvic therapy, until at least one day after the pelvic therapy. For example, prior to the first dose of radiotherapy, chemotherapy, and/or prior to undergoing the surgical procedure

According to another aspect, the method of protecting against radiation induced enteritis includes administering to a subject in need thereof a therapeutically effective amount of balsalazide.

In another aspect, the method of protecting against radiation induced injury to the mucosa of the colon includes administering to a subject in need thereof a therapeutically effective amount of balsalazide.

In yet another aspect, the method of protecting against radiation induced colorectal inflammation includes administering to a subject in need thereof a therapeutically effective amount of balsalazide.

Other embodiments of the invention are disclosed infra.

### DESCRIPTION OF THE DRAWINGS

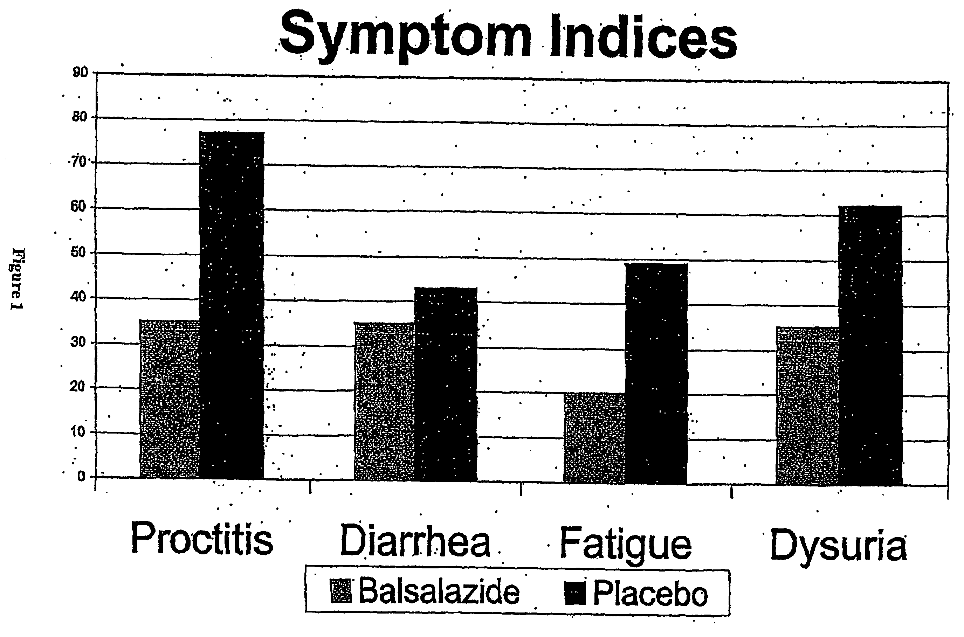

Fig. 1 shows the symptom index for balsalazide treated patients in accordance with one embodiment of the present invention and placebo treated patients.

### DETAILED DESCRIPTION OF THE INVENTION

Disclosed herein are methods of treating radiation induced enteritis.

Radiation therapy and radiotherapy are used interchangeably herein and include external irradiation and internal irradiation, also referred to as brachytherapy, intracavitary brachytherapy, or interstitial brachytherapy. Radiation sources contemplated include pure Gamma, pure Beta and mixed irradiations.

As used herein, the terms "chemotherapy" and "chemotherapeutic agents" are used interchangeably and refer to chemotherapeutic agents or drugs exhibiting anti-cancer effects and used in the treatment of malignancies.

We have surprisingly found that the administration of balsalazide to a patient experiencing radiation induced enteritis, reduces symptoms of the condition. The success of balsalazide in the treatment of radiation enteritis is surprising because of the failure of other related 5-ASA drugs, such as olsalazine and mesalamine, in clinical trials.

Balsalazide is the generic name for a 2-hydroxy-5-phenylazobenzoic acid derivative in which an aminosalicylate moiety, 5-aminosalicylic acid (5-ASA) (mesalamine), is linked to a carrier molecule, 4-aminobenzoyl-*-alanine (4-ABA), through an azo-bond. Disodium balsalazide is highly water-soluble and is cleaved in the colon to release mesalamine, which is the therapeutically active portion of the molecule, as well as 4-aminobenzol-alamine, which is the carrier moiety. Mesalamine is 5-aminosaliacylic acid and appears to act topically.

The use of balsalazide to treat radiation induced enteritis is especially beneficial because is metabolized by intestinal microflora to the active form, 5-ASA, thus ensuring optimal delivery of the active drug to the bowel without loss via absorption more proximally in the intestinal tract. Balsalazide also exhibits fewer side effects than other 5-ASA prodrugs and it may be administered to subjects with sulpha allergies. Balsalazide is also beneficial because the active component has been demonstrated to directly scavenge free radicals, which may reduce subsequent inflammatory response. Without wishing to be bound by any particular theory, we believe that balsalazide may protect against radiation-induced enteritis by blocking the mediators of inflammation and the release of free radicals in the rectal mucosa.

As used herein radiation induced enteritis includes radiation induced injury to the pelvic area from irradiation of the pelvic region. Irradiation often causes acute radiation enteritis or colorectal toxicity. Symptoms may include diarrhea, proctitis, stool incontinence, loose stool, increased defecations per day, tenesmus, mucous production, abdominopelvic pain, and peri-rectal discomfort. Acute radiation enteritis results largely from irritation of the sigmoid colon and rectum.

Yet another aspect of this invention relates to a use of balsalazide for treating a subject (e.g., mammal, human, horse, dog, cat) who is in need thereof. Identifying a subject in need of such treatment can be in the judgment of a subject or a health care professional and can be subjective (e.g. opinion) or objective (e.g. measurable by a test or diagnostic method).

Balsalazide may be used in various treatment regimes. These regimes may vary depending upon the subject and the type of treatment.

Balsalazide may be administered prior to, during, and/or after the treatment therapies. Balsalazide may be administered, for example, twice a day, three times a day, or four times a day. Balsalazide may be administered in doses, for example of from about between 2000 mg BID to about 2500 mg TID. Another example is administering balsalazide from between about 4.0 g/day to about 7.25 g/day. The balsalazide may be administered, for example, in tablet form, powered form, liquid for or in capsules.

Subjects in need thereof are subjects that will undergo radiation therapy, either alone or in combination with other pelvic therapies that could induce enteritis. This need may be apparent prior to undergoing radiation therapy either alone or in combination with chemotherapy of a pelvic surgical procedure; for subjects undergoing radiation therapy either alone or in combination with chemotherapy or a pelvic surgical procedure and for subjects post radiation therapy either alone or in combination with chemotherapy or a pelvic surgical procedure. For example, subjects may be about to undergo, may be undergoing, or have undergone radiation therapy in combination with chemotherapy or a surgical procedure.

Also included are subjects who are or who may be susceptible to enteritis whereby the enteritis is radiation induced enteritis and it is caused by radiation therapy alone or in combination with chemotherapy or a surgical procedure. Subjects may be suffering from, for example, gastrointestinal malignancies, including colorectal, appendiceal, anal, or small bowel cancers; urogenital malignancies, including prostate, bladder, testicular, or penile cancers; gynecologic malignancies, including cervical, endometrial, ovarian, vaginal, or vulvar cancers; or osteogenic and other sarcomatous malignancies in which pelvic structures are involved..

As used herein, a therapeutically effective amount means an amount effective, when administered to a human or non-human subject, to provide a therapeutic benefit such as an amelioration of symptoms, e.g., an amount effective to decrease the symptoms of acute radiation enteritis.

According to certain embodiments, balsalazide may be administered prior to radiotherapy. Balsalazide may be administered, for example, at least one day prior to the subject's first dose of radiotherapy, at least five days prior to the subject's first dose of radiotherapy, during radiation therapy, for at least one day after the cessation of radiation therapy, for fourteen days after the cessation of radiation therapy. Administration at least five days prior to the therapy includes administration daily, every day prior to the pelvic therapy, administration on a majority of days prior the therapy, administration on the day of treatment or no administration on the day of treatment.

Certain preferred embodiments include administering balsalazide from at least one day prior to the first dose of radiotherapy until at least one day after the cessation of radiation therapy. Treatment prior to the radiation therapy allows for the 5-ASA to be present at its site of action during the cause of the injury.

In certain embodiments, the balsalazide is administered to a subject from between about 2 weeks to about 6 weeks in duration, from between about 8 weeks to about 12 weeks in duration, or from between 1 day to about 7 days. The balsalazide may be administered intermittently or continuously during the course of treatment. Length of treatment may vary depending of the type and length of radiotherapy, chemotherapy, and/or type of surgical procedure and the proper length of treatment may be easily determined by one of skill in the art having the benefit of this disclosure.

For any of the embodiments, balsalazide may be administered, for example, once daily, twice daily, three times daily, or four times daily to a subject. In some particularly preferred methods of the present invention comprise administering the balsalazide twice daily to the subject because it may, for example, minimize the side effects and increase patient compliance.

Dosages, according to certain preferred embodiments, range from between about 3,000 mg to about 5000 mg of balsalazide administered daily. For example, a dose of 2250 mg may be administered to a subject twice daily. Other appropriate dosages for methods according to this invention may be determined by health care professionals or by the subject. The amount of balsalazide administered daily may be increased or decreased based on the weight, age, health, sex or medical condition of the subject. One of skill in the art would be able to determine the proper dose for a subject based on this disclosure.

For subjects undergoing multiple therapies, whereby radiotherapy is performed either alone or in combination with chemotherapy or a surgical procedure balsalazide may be administered, for example, at least one day prior to the subject's first dose of radiotherapy, chemotherapy or prior to undergoing a surgical procedure; at least five days prior to the subject's first dose of radiotherapy, chemotherapy or prior to undergoing a surgical procedure; during radiation therapy, chemotherapy or the surgical procedure; at least one day after the cessation of radiation therapy, chemotherapy, or after the surgical procedure; for fourteen days after the cessation of radiation therapy, chemotherapy, or after the surgical procedure.

It is often preferable to administer the balsalazide to a subject prior to treatment, during treatment, as well as after the cessation of treatment. For example, balsalazide may be administered from at least one day prior to the first dose of radiotherapy, chemotherapy, and/or prior to undergoing the surgical procedure until at least one day after the cessation of radiation therapy, chemotherapy, or the surgical procedure.

Indications include a subject receiving radiotherapy either alone or in combination with chemotherapy or surgical procedure as a result of treatment for cancer of the cervix, prostate, appendix, colon, intestine, rectum, or other gastrointestinal malignancy, or prostatectomy.

According to certain embodiments, balsalazide may be administered in combination with other compounds, including for example, chemotherapeutic agents, anti-inflammatory agents, anti-pyretic agents radiosensitizing agents, radioprotective agents, urologic agents, anti-emetic agents, and/or anti-diarrheal agents. For example, cisplatin, carboplatin, docetaxel, paclitaxel, flurouracil, capecitabine, gemcitabine, irinotecan, topotecan, etoposide, mitomycin, gefitinib, vincristine, vinblastine, doxorubicin, cyclophosphamide, celecoxib, rofecoxib, valdecoxib, ibuprofen, naproxen, ketoprofen, dexamethasone, prednisone, prednisolone, hydrocortisone, acetaminophen, misonidazole, amifostine, tamsulosin, phenazopyridine, ondansetron, granisetron, alosetron, palonosetron, promethazine, prochlorperazine, trimethobenzamide, aprepitant, diphenoxylate with atropine, and/or loperamide.

The uses disclosed herein are also useful for protecting a subject against radiation induced enteritis by administering to a subject in need thereof a therapeutically effective amount of balsalazide. For example, prophylactic doses may be administered prior to a patient undergoing radiation.

The methods disclosed herein are useful for protecting a subject against radiation induced injury to the mucosa of the colon, as well as against radiation induced colorectal inflammation by administering to a subject in need thereof a therapeutically effective amount of balsalazide.

### Examples

It should be appreciated that the invention should not be construed to be limited to the example, which is now described; rather, the invention should be construed to include any and all applications provided herein and all equivalent variations within the skill of the ordinary artisan.

### Clinical Trial of Balsalazide to Treat Radiation Enteritis

Subjects included patients treated for FIGO stage IB2-IVA cervical cancer, AJCC Stage T1-3 MO prostate cancer, or biochemical failure after prostatectomy. Radiotherapy was delivered to the subjects in a 4-field technique to at least 40 Gy, and tumor dose was at least 64 Gy for prostate patients, and 75 Gy for cervical patients. Brachytherapy boost was permitted.

Patients were given 2250 mg of BSZ or an identical-appearing placebo twice daily beginning 5 days prior to radiotherapy, and continuing for 2 weeks after completion. Toxicities were graded weekly according to NCI Common Toxicity Criteria for each of the following: proctitis, diarrhea, dysuria, weight loss, fatigue, nausea, and vomiting. A symptom index was formulated for each toxicity consisting of the toxicity's numeric grade multiplied by the number of days it was experienced, and summed for each grade. Thus, a patient with 7 days of grade 1 proctitis, 14 days of grade 2 proctitis, and 7 days of grade 3 proctitis [(1*7)+ (2*14)+(3*7)] would have a proctitis index of 56. A higher Index indicates worse toxicity.

Patients were randomized by sealed envelope decision, with only the protocol coordinator aware of the results of randomization. After randomization, the study drug or identical-appearing placebo was given to the study subject with instructions concerning dosage and administration schedule.

As stated above, the toxicity was assessed weekly during the course of radiation treatment, then for a period of at least six months after completion of radiotherapy. National Cancer Institute (NCI) common toxicity criteria were used to grade the severity of radiation-induced acute bowel toxicity. Diarrhea was used as the primary endpoint and was assessed as follows:
Grade 0 - no diarrhea or increased stool frequency
Grade 1 - increase of 2-3 stools/day
Grade 2 - increase of 4-6 stools/day or nocturnal stool
Grade 3 - increase of 7-9 stools/day or incontinence
Grade 4 - increase of > 10 stools/day or grossly bloody diarrhea

Documented baseline hemorrhoidal bleeding was not considered grade 4.

A secondary endpoint included proctitis, which was assessed using guidelines outlined by the NCI Common Toxicity Criteria, including:
Grade 0 - None
Grade 1 - increased stool frequency, occasional blood-streaked stools or rectal discomfort, not requiring medication
Grade 2 - increased stool frequency, bleeding, mucous discharge or rectal discomfort requiring medication
Grade 3 - increased stool frequency/diarrhea requiring parenteral support, rectal bleeding requiring transfusion, or persistent mucus discharge necessitating the use of pads
Grade 4 - perforation, bleeding or necrosis, or other life-threatening complications requiring surgical intervention

Clinical experience indicated that the only statistically significant side effect of balsalazide disodium was abdominal pain, which is seen in 11% of patients receiving the drug versus 0% in those receiving placebo. Adverse events reported in trials of balsalazide were comparable to those seen in patients receiving placebo, which included headache, nausea, diarrhea, flatulence, and fatigue.

Except for the nausea/vomiting seen in 2 patients on balsalazide and 1 on placebo, all toxicities were appreciably lower in patients taking balsalazide. Proctitis was prevented most effectively, with a mean index of 35 in balsalazide patients vs. 77 in placebo patients (p=0.04). No patient on balsalazide experienced grade 4 diarrhea, while 2 placebo patients did. Average weight loss in placebo patients was greater than 3 pounds, whereas average balsalazide patients gained weight. Fatigue index in balsalazide patients was 20, while placebo patients averaged 49. Unexpectedly, dysuria was appreciably lower in balsalazide patients as well. Figure 1 is a bar graph presenting the symptom indices comparing subjects administered balsalazide and those given placebo.

## Claims

1. Balsalazide for use as a medicament for treating radiation induced enteritis in a subject in need thereof.

2. Balsalazide of claim 1, wherein the balsalazide is administered at least one day prior to the subject's first dose of radiotherapy.

3. Balsalazide of claim 1, wherein the balsalazide is administered at least five days prior to the subject's first dose of radiotherapy.

4. Balsalazide of claim 1, wherein the balsalazide is administered during radiation therapy.

5. Balsalazide of claim 1, wherein the balsalazide is administered for at least one day after the cessation of radiation therapy.

6. Balsalazide of claim 1, wherein the balsalazide is administered for fourteen days after the cessation of radiation therapy.

7. Balsalazide of claim 1, wherein the balsalazide is administered from at least one day prior to the first dose of radiotherapy until at least one day after the cessation of radiation therapy.

8. Balsalazide of claim 1, wherein the subject received radiotherapy as a result of treatment for gastrointestinal malignancies, including colorectal, appendiceal, anal, or small bowel cancers; urogenital malignancies, including prostate, bladder, testicular, or penile cancers; gynecologic malignancies, including cervical, endometrial, ovarian, vaginal, or vulvar cancers; or osteogenic and other sarcomatous malignancies in which pelvic structures are involved.

9. Balsalazide of claim 1, wherein the balsalazide is administered twice daily to the subject.

10. Balsalazide of claim 1, wherein from between about 3,000mg to about 5000mg of balsalazide is administered to the subject daily.

11. Balsalazide of claim 1, wherein the balsalazide is administered to the subject from between about 8 weeks to about 12 weeks.

12. Balsalazide of claim 1, wherein the subject has acute radiation enteritis.

13. Balsalazide of claim 1, wherein the.radiation induced enteritis is caused by radiation therapy in combination with chemotherapy or a surgical procedure.

14. Balsalazide of claim 13, wherein the balsalazide is administered at least one day prior to the subject's first dose of radiotherapy, chemotherapy, or prior to undergoing a surgical procedure.

15. Balsalazide of claim 13, wherein the balsalazide is administered at least five days prior to the subject's first dose of radiotherapy, chemotherapy, or prior to undergoing a surgical procedure.

16. Balsalazide of claim 13, wherein the balsalazide is administered during radiation therapy, chemotherapy, or the surgical procedure.

17. Balsalazide of claim 13, wherein the balsalazide is administered for at least one day after the cessation of radiation therapy, chemotherapy, or after the surgical procedure.

18. Balsalazide of claim 13, wherein the balsalazide is administered for fourteen days after the cessation of radiation therapy, chemotherapy, or after the surgical procedure.

19. Balsalazide of claim 13, wherein the balsalazide is administered from at least one day prior to the first dose of radiotherapy, chemotherapy, or prior to undergoing the surgical procedure until at least one day after the cessation of radiation therapy, chemotherapy, or the surgical procedure.

20. Balsalazide of claim 13, wherein the subject received radiotherapy, chemotherapy, or surgical procedure as a result of treatment for gastrointestinal malignancies, including colorectal, appendiceal, anal, or small bowel cancers; urogenital malignancies, including prostate, bladder, testicular, or penile cancers; gynecologic malignancies, including cervical, endometrial, ovarian, vaginal, or vulvar cancers; or osteogenic and other sarcomatous malignancies in which pelvic structures are involved.

21. Balsalazide of claim 13, wherein the balsalazide is administered twice daily to the subject.

22. Balsalazide of claim 13, wherein from between about 3,000mg to about 5000mg of balsalazide is administered to the subject daily.

23. Balsalazide of claim 13, wherein the balsalazide is administered to the subject from between about 8 weeks to about 12 weeks.

24. Balsalazide for use as a medicament for protecting against radiation induced enteritis or for protecting against radiation induced injury to the mucosa of the colon, or for protecting against radiation induced colorectal inflammation, wherein the balsalazide is administered to a subject before, during, and/or after undergoing radiation therapy.

25. Use of balsalazide for the manufacture of a medicament for treating radiation induced enteritis in a subject in need thereof.

## Patentansprüche

1. Balsalazid zur Verwendung als Medikament für eine Behandlung strahleninduzierter Enteritis bei einem Subjekt, das Bedarf dafür hat.

2. Balsalazid nach Anspruch 1, wobei das Balsalazid wenigstens einen Tag vor der ersten Strahlentherapie-Dosis des Subjekts verabreicht wird.

3. Balsalazid nach Anspruch 1, wobei das Balsalazid wenigstens fünf Tage vor der ersten Strahlentherapie-Dosis des Subjekts verabreicht wird.

4. Balsalazid nach Anspruch 1, wobei das Balsalazid während einer Strahlentherapie verabreicht wird.

5. Balsalazid nach Anspruch 1, wobei das Balsalazid für wenigstens einen Tag nach Beendigung der Strahlentherapie verabreicht wird.

6. Balsalazid nach Anspruch 1, wobei das Balsalazid für 14 Tage nach Beendigung der Strahlentherapie verabreicht wird.

7. Balsalazid nach Anspruch 1, wobei das Balsalazid ab wenigstens einem Tag vor der ersten Strahlentherapie-Dosis bis wenigstens einen Tag nach Beendigung der Strahlentherapie verabreicht wird.

8. Balsalazid nach Anspruch 1, wobei das Subjekt Strahlentherapie als Folge einer Behandlung von gastrointestinalen Malignitäten, einschließlich Kolorektal-, Appendix-, Anal- oder Dünndarmkrebs; urogenitalen Malignitäten, einschließlich Prostata-, Blasen-, Hoden- oder Peniskrebs; gynäkologischen Malignitäten, einschließlich zervikalem, endometrialem, ovarialem, vaginalem oder vulvarem Krebs, oder osteogenen und anderen sarkomatösen Malignitäten, bei denen Beckenstrukturen involviert sind, erhält bzw. erhielt.

9. Balsalazid nach Anspruch 1, wobei das Balsalazid zweimal täglich an das Subjekt verabreicht wird.

10. Balsalazid nach Anspruch 1, wobei zwischen etwa 3.000 mg und etwa 5.000 mg Balsalazid täglich an das Subjekt verabreicht werden.

11. Balsalazid nach Anspruch 1, wobei das Balsalazid zwischen etwa 8 Wochen und etwa 12 Wochen an das Subjekt verabreicht wird.

12. Balsalazid nach Anspruch 1, wobei das Subjekt akute Strahlungsenteritis hat.

13. Balsalazid nach Anspruch 1, wobei die strahlungsinduzierte Enteritis durch Strahlentherapie in Kombination mit Chemotherapie oder einem chirurgischen Eingriff verursacht ist.

14. Balsalazid nach Anspruch 13, wobei das Balsalazid wenigstens einen Tag vor der ersten Strahlentherapie-Dosis, der Chemotherapie des Subjekts oder vor Unterziehen einem chirurgischen Verfahren verabreicht wird.

15. Balsalazid nach Anspruch 13, wobei das Balsalazid wenigstens fünf Tage vor der ersten Strahlendosis, der Chemotherapie des Subjekts oder vor Unterziehen einem chirurgischen Eingriff verabreicht wird.

16. Balsalazid nach Anspruch 13, wobei das Balsalazid während einer Strahlentherapie, Chemotherapie oder des chirurgischen Eingriffs verabreicht wird.

17. Balsalazid nach Anspruch 13, wobei das Balsalazid für wenigstens einen Tag nach Beendigung der Strahlentherapie, Chemotherapie oder nach dem chirurgischen Eingriff verabreicht wird.

18. Balsalazid nach Anspruch 13, wobei das Balsalazid für 14 Tage nach Beendigung der Strahlentherapie, der Chemotherapie oder nach dem chirurgischen Eingriff verabreicht wird.

19. Balsalazid nach Anspruch 13, wobei das Balsalazid ab wenigstens einem Tag vor der ersten Strahlentherapie, Chemotherapie oder vor Unterziehen dem chirurgischen Eingriff bis wenigstens einen Tag nach der Beendigung der Strahlentherapie, der Chemotherapie oder des chirurgischen Eingriffs verabreicht wird.

20. Balsalazid nach Anspruch 13, wobei das Subjekt Strahlentherapie, Chemotherapie oder einen chirurgischen Eingriff als Folge einer Behandlung von gastrointestinalen Malignitäten, einschließlich Kolorektal-, Appendix-, Anal- oder Dünndarmkrebs; urogenitalen Malignitäten, einschließlich Prostata-, Blasen-, Hoden- oder Peniskrebs; gynäkologischen Malignitäten, einschließlich zervikalem, endometrialem, ovarialem, vaginalem oder vulvarem Krebs, oder osteogenen und anderen sarkomatösen Malignitäten, bei denen Beckenstrukturen involviert sind, erhält bzw. erhielt.

21. Balsalazid nach Anspruch 13, wobei das Balsalazid zweimal täglich an das Subjekt verabreicht wird.

22. Balsalazid nach Anspruch 13, wobei zwischen etwa 3.000 mg und etwa 5.000 mg Balsalazid täglich an das Subjekt verabreicht werden.

23. Balsalazid nach Anspruch 13, wobei das Balsalazid zwischen etwa 8 Wochen und etwa 12 Wochen an das Subjekt verabreicht wird.

24. Balsalazid zur Verwendung als Medikament zum Schutz gegen strahlungsinduzierte Enteritis oder zum Schutz gegen strahlungsinduzierte Verletzung an der Mukosa des Kolons oder zum Schutz gegen strahlungsinduzierte kolorektale Entzündung, wobei das Balsalazid vor, während und/oder nach Unterziehung einer Strahlentherapie an ein Subjekt verabreicht wird.

25. Verwendung von Balsalazid für die Herstellung eines Medikaments zur Behandlung von strahlungsinduzierter Enteritis bei einem Subjekt, das Bedarf dafür hat.

## Revendications

1. Balsalazide pour l'utilisation comme médicament pour traiter l'entérite radio-induite chez un sujet qui en a besoin.

2. Balsalazide selon la revendication 1, le balsalazide étant administré au moins un jour avant la première dose de radiothérapie du sujet.

3. Balsalazide selon la revendication 1, le balsalazide étant administré au moins cinq jours avant la première dose de radiothérapie du sujet.

4. Balsalazide selon la revendication 1, le balsalazide étant administré pendant la radiothérapie.

5. Balsalazide selon la revendication 1, le balsalazide étant administré pour au moins un jour après la cessation de la radiothérapie.

6. Balsalazide selon la revendication 1, le balsalazide étant administré pour quatorze jours après la cessation de la radiothérapie.

7. Balsalazide selon la revendication 1, le balsalazide étant administré commençant au moins un jour avant la première dose de radiothérapie jusqu'à au moins un jour après la cessation de la radiothérapie.

8. Balsalazide selon la revendication 1, où le sujet reçoit la radiothérapie à la suite d'un traitement des malignités gastro-intestinales, y compris le cancer colorectal, le cancer d'appendice, le cancer anal ou le cancer de l'intestine grêle; des malignités urogénitales, y compris le cancer de la prostate, le cancer de la vessie, le cancer testiculaire ou le cancer du pénis; des malignités gynécologiques, y compris le cancer cervical, le cancer de l'endométriome, le cancer ovarien, le cancer vaginal ou le cancer vulvaire; ou des malignités ostéogènes et des autres malignités sarcomateuses, chez lesquelles des structures pelviennes sont impliquées.

9. Balsalazide selon la revendication 1, le balsalazide étant administré deux fois par jour.

10. Balsalazide selon la revendication 1, où d'environ 3000 mg à environ 5000 mg de balsalazide sont administrés quotidiennement au sujet.

11. Balsalazide selon la revendication 1, où le balsalazide est administré au sujet d'environ 8 semaines à environ 12 semaines.

12. Balsalazide selon la revendication 1, où le sujet souffre de l'entérite radio-induite aïgue.

13. Balsalazide selon la revendication 1, où l'entérite radio-induite est provoquée par radiothérapie combinée à une chimiothérapie ou une intervention chirurgicale.

14. Balsalazide selon la revendication 13, où le balsalazide est administré au moins un jour avant la première dose de la radiothérapie, de la chimiothérapie du sujet ou avant une intervention chirurgicale.

15. Balsalazide selon la revendication 13, où le balsalazide est administré au moins cinq jours avant la première dose de la radiothérapie, de la chimiothérapie du sujet ou avant une intervention chirurgicale.

16. Balsalazide selon la revendication 13, où le balsalazide est administré pendant la radiothérapie, la chimiothérapie ou l'intervention chirurgicale.

17. Balsalazide selon la revendication 13, où le balsalazide est administré pour au moins un jour après la cessation de la radiothérapie, de la chimiothérapie ou après l'intervention chirurgicale.

18. Balsalazide selon la revendication 13, où le balsalazide est administré pour quatorze jours après la cessation de la radiothérapie, de la chimiothérapie ou de l'intervention chirurgicale.

19. Balsalazide selon la revendication 13, où le balsalazide est administré commençant au moins un jour avant la première dose de la radiothérapie, de la chimiothérapie ou avant l'intervention chirurgicale jusqu'à au moins un jour après la cessation de la radiothérapie, de la chimiothérapie ou de l'intervention chirurgicale.

20. Balsalazide selon la revendication 13, où le sujet recevait la radiothérapie, la chimiothérapie ou l'intervention chirurgicale à la suite d'un traitement de malignités gastro-intestinales, y compris le cancer colorectal, le cancer d'appendice, le cancer anal ou le cancer de l'intestine grêle; des malignités urogénitales, y compris le cancer de la prostate, le cancer de la vessie, le cancer testiculaire ou le cancer du pénis; des malignités gynécologiques, y compris le cancer cervical, le cancer de l'endométriome, le cancer ovarien, le cancer vaginal ou le cancer vulvaire; ou des malignités ostéogènes et des autres malignités sarcomateuses, chez lesquelles des structures pelviennes sont impliquées.

21. Balsalazide selon la revendication 13, où le balsalazide est administré deux fois par jour.

22. Balsalazide selon la revendication 13, où d'environ 3000 mg à environ 5000 mg de balsalazide sont administrés quotidiennement au sujet.

23. Balsalazide selon la revendication 13, le balsalazide est administré au sujet d'environ 8 semaines à environ 12 semaines.

24. Balsalazide pour l'utilisation comme médicament pour protéger contre une entérite radio-induite ou pour protéger contre une blessure radio-induite de la muqueuse du côlon ou pour protéger contre une inflammation colorectale radio-induite, où le balsalazide est administré à un sujet avant, pendant et/ou après une radiothérapie.

25. Utilisation du balsalazide pour la fabrication d'un médicament destiné au traitement de l'entérite radio-induite chez un sujet qui en a besoin.
